# EUROPEAN PATENT APPLICATION

(11) **EP 3 095 880 A1**
(43) Date of publication of application: **23.11.2016**
(21) Application number: 15168354.7
(22) Date of filing: 20.05.2015
(51) Int. Cl.: C12R 1/46, C12R 1/77, G01N 33/569, C12R 1/66

(54) **METHOD FOR THE DIAGNOSIS OF FUSARIUM INFECTIONS**

(71) Applicant: Ludwig-Maximilians-Universität München, 80539 München (DE)
(72) Inventor: Ebel, Frank, 80689 Munich (DE); Wiedemann, Annegret, 80336 Munich (DE)
(74) Representative: Kopf Westenberger Wachenhausen Patentanwälte PartG mbB

(57) **Abstract**

The present invention relates to an antibody or functional fragment thereof, which specifically binds to a galactofuranose Gal*f* antigen present in *Fusarium spec.,* said antigen being distinct from the β-1,5-Gal*f*ₙ antigen. The invention further relates to the use of such antibody for the diagnosis of fungal infections caused by *Fusarium spec.* and for the differentiation of *Fusarium spec.* from *Aspergillus spec.*

## Description

### FIELD OF THE INVENTION

The present invention relates to the diagnosis of fungal infections caused by *Fusarium spec.* More particularly, the invention relates to an antibody, which specifically binds to a galactofuranose Gal*f* antigen that is distinct from the β-1,5-Gal*f*ₙ antigen, thus enabling a differentiation between *Fusarium* and *Aspergillus.*

### BACKGROUND OF THE INVENTION

Diagnostics of invasive fungal infections relies currently on culture, histology or detection of circulating cell wall carbohydrates (De Pauw, B. et al. (2008) Clin. Infect. Dis. 46, 1813-1821). Two major antigens of filamentous fungi can be detected by serological assays: galactomannan and β-D-glucan. However, β-D-glucan is a common cell wall component, and thus not indicative for a specific pathogen. In contrast, galactomannan is only produced by few fungal pathogens, such as *Aspergillus* and *Penicillium* (Swanink, C.M. et al. (1997) J. Clin. Microbiol. 35, 257-260).

*Fusarium* is the second most frequent mold causing infections in humans. The most relevant pathogenic species are *F. verticilloides* and *F. proliferatum* (Nucci, M. and Anaissie, E. (2007) Clin. Microbiol. Rev. 20, 695-704).

*Fusarium* and *Aspergillus* differ in their sensitivity to commonly used anti-fungal agents, such as echinocandines (Alastruey-Izquierdo, A. et al. (2008) J. Antimicrob. Chemother. 61, 805-809). A precise differentiation of these two major fungal infections is desirable for efficient therapy. However, differentiation by conventional histology is hampered by similar hyphal morphologies, and the β-D-glucan assay is positive for both *Fusarium* and *Aspergillus.* On the other hand, galactomannan has been identified as characteristic cell wall component of *Aspergillus.* A corresponding galactomannan assay (commercially available from Bio-Rad Laboratories as PLATELIA *Aspergillus* EIA kit) and its key component, antibody EB-A2, were originally reported to discriminate between *Aspergillus* and *Fusarium* (Stynen, D. et al. (1992) Infect. Immun. 60, 2237-2245; Cummings, J.R. et al. (2007) Diagn. Microbiol. Infect. Dis. 59, 113-115).

A fungal infection associated with a positive β-D-glucan, but a negative galactomannan assay is likely to be a *Fusarium* infection (Nucci, M. and Anaissie, E. (2007), *supra).* However, the galactomannan assay does not allow for an accurate and reliable differentiation of *Fusarium* and *Aspergillus* infections. Recently, several studies reported an association between *Fusarium* infections and a positive galactomannan assay (Mikulska, M. et al. (2012) Diagn. Microbiol. Infect. Dis. 72, 367-369; Horn, D.L.et al. (2014) Mycoses 57, 652-658; Kebabci, N. et al. (2014) Mycoses 57, 249-255; Nucci, M. et al. (2014) PLoS One 9, e87784). Furthermore, galactomannan antigens were detected in culture supernatants of the corresponding *Fusarium* isolates (Tortorano, A.M. et al. (2012) J. Clin. Microbiol. 50, 1051-1053). These data suggest that *Fusarium* species can produce antigens that are recognized by antibody EB-A2 used in the galactomannan assay.

Fungal galactomannan includes galactofuranose (Gal*f*) as characteristic component, while galactomannan from other sources (e.g., plants) includes galactopyranose. The rat monoclonal antibody EB-A2 employed in the galactomannan assay was shown to bind to chains of synthetic β-1,5-linked Galf (Stynen, D. et al. (1992), *supra)* indicating a specificity for β-1,5-Gal*f*ₙ that was shown to be present in several *A. fumigatus* cell wall glycostructures (Tefsen, B. et al. (2012) Glycobiology 22, 456-469).

Synthesis of Gal*f*-containing structures consists of at least three steps: synthesis of UDP-bound Gal*f*, transport of UDP-Gal*f* into the Golgi apparatus, and incorporation of Gal*f* into glycostructures by a set of UDP-galactofuranosyl-transferases (Tefsen et al. (2011), *supra).* In *A. fumigatus,* the first step is mediated by the UDP-Gal*f* mutase GlfA (Schmalhorst, P.S. et al. (2008) Eukaryot. Cell 7, 1268-1277), while the UDP-Gal*f* transporter GlfB is required for the second step (Engel, J. et al. (2009) J. Biol. Chem. 284, 33859-33868). Since several types of Gal*f* linkages exist in *A. fumigatus,* it is conceivable that a set of UDP-galactofuranosyl-transferases exist, but up to date only one has been identified (Komachi, Y. et al. (2013) Mol. Microbiol. 90, 1054-1073). So far, no corresponding proteins have been identified in *Fusarium.*

Thus, there is still a need for molecular tools, in particular specific antibody molecules, as well as corresponding detection methods in order to enable an easy and accurate identification of *Fusarium* and its differentiation from *Aspergillus.* Such molecular tools and methods would be of utmost clinical importance for the reliable diagnosis of fungal infections caused by *Fusarium,* thus overcoming the above limitations of existing diagnostic assays.

Accordingly, it is an object of the present invention to provide such molecular tools and corresponding methods for the diagnosis of *Fusarium* infections.

### SUMMARY OF THE INVENTION

In one aspect, the present invention relates to an antibody or functional fragment thereof, which specifically binds to a galactofuranose Gal*f* antigen present in *Fusarium spec.,* wherein the antigen is distinct from the β-1,5-Gal*f*ₙ antigen.

In a specific embodiment, the antibody has less than 5% cross-reactivity to the β-1,5-Gal*f*ₙ antigen. Preferably, the antibody is a monoclonal antibody. It is also preferred that the antibody is an IgM antibody.

In a particularly preferred embodiment, the antibody is monoclonal antibody AB135-8 being produced by the hybridoma cell line having accession number DSM ACC3270.

Another aspect of the invention relates to the use of the antibody or functional fragment thereof as defined herein for the diagnosis of fungal infections caused by *Fusarium spec.*

Yet another aspect of the present invention relates to the use of the galactofuranose Gal*f* antigen being specifically recognized by the antibody or functional fragment thereof as defined herein as biomarker for diagnosing a fungal infection caused by *Fusarium spec.*

In a further aspect, the present invention relates to a method for the identification of *Fusarium spec.* in a sample, comprising: (i) contacting the antibody or functional fragment thereof as defined herein with a sample supposed to comprise *Fusarium spec.;* and (ii) determining the binding of the antibody or functional fragment thereof to the corresponding galactofuranose Gal*f* antigen, wherein detection of binding of the antibody is indicative for the presence of *Fusarium spec.* in the sample.

In a specific embodiment, the method further comprises: (iii) quantifying the level of antibody binding in the sample and comparing to a control level.

In a specific embodiment, the method further comprises: (iv) contacting the sample with a second antibody or a functional fragment thereof, wherein the second antibody is capable of specifically binding to the β-1,5-Gal*f*ₙ antigen; and (v) determining the binding of the second antibody or functional fragment thereof to the corresponding β-1,5-Gal*f*ₙ antigen, wherein detection of binding of the second antibody is indicative for the presence of *Aspergillus spec.* in the sample. Preferably, the second antibody is selected from the group consisting of antibody L10-1 and antibody EB-A2.

In a preferred embodiment, antibody binding is determined by immunofluorescence microscopy or enzyme-linked immunosorbent assay.

In yet another aspect, the invention relates to a kit-of parts, comprising: (i) a first antibody or functional fragment thereof as defined herein; and (ii) a second antibody being capable of specifically binding to the β-1,5-Gal*f*ₙ antigen. Preferably, the second antibody is selected from the group consisting of antibody L10-1 and antibody EB-A2.

In a preferred embodiment, the kit-of-parts is used in a diagnostic assay for differentiating *Fusarium spec.* from *Aspergillus spec.*

### BRIEF DESCRIPTION OF THE DRAWINGS

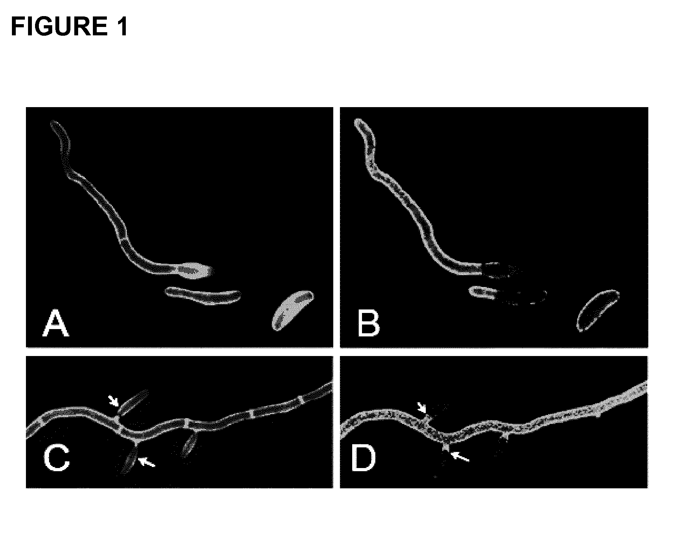

**FIGURE 1****: Monoclonal antibody AB135-8 recognizes hyphae and conidia (spores) of *Fusarium proliferatum.***
   Hyphae of *F. proliferatum* were grown in YG medium. After fixation, samples were stained with calcofluor white (panels A and C) and incubated with AB135-8, followed by immunofluorescence detection (panels B and D), respectively. All panels show projections of several optical planes. Arrows in D indicate AB135-8-labeled cup-like structures connecting conidia and hyphae.

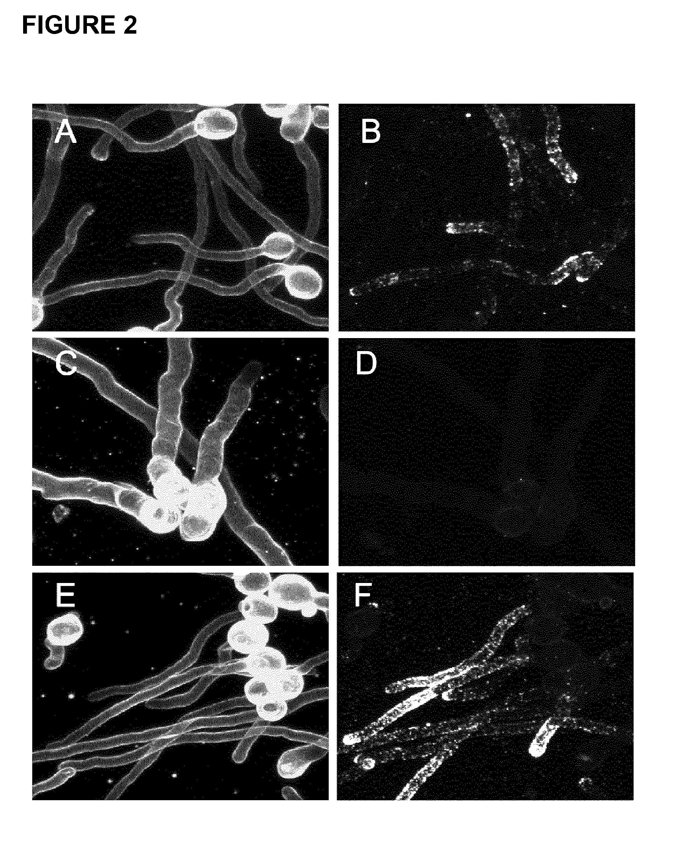

**FIGURE 2****: Labeling of *Aspergillus fumigatus* hyphae by AB135-8 depends on Gal*f*.**
   Hyphae of *A. fumigatus* wild type strain D141 (panels A and B), of the Δ*glfA* mutant (panels C and D), and the complemented mutant (panels E and F) were stained with calcofluor white (panels A, C and E) and incubated with AB135-8, followed by immunofluorescence detection (panels B, D and F), respectively. Notably, AB135-8 is not recognizing hyphae of the Δ*glfA* mutant lacking Galf.

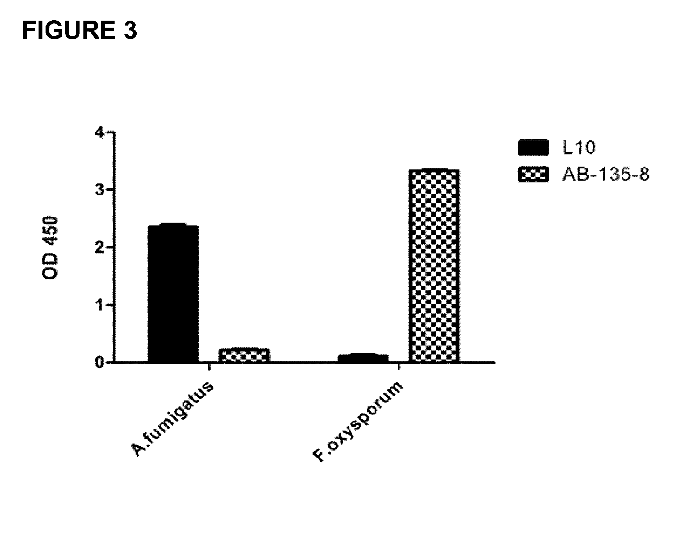

**FIGURE 3****: Detection of galactomannan (β-1,5-Gal*f*ₙ) antigen and the "AB135-8 Gal*f*" antigen in culture supernatants.**
   Cell-free supernatants of *Aspergillus fumigatus* and *Fusarium oxysporum* were diluted 1:100 in coating buffer and coated onto ELISA plates. Plates were blocked and incubated with the antibodies L10-1 (functionally equivalent to EB-A2) and AB135-8, respectively. After further washing, wells were incubated with peroxidase-labelled anti-mouse IgM antibody. Enzymatic activity was detected by determining optical density at 450 nm. Experiments were performed in triplicate and shown as mean ± SEM.

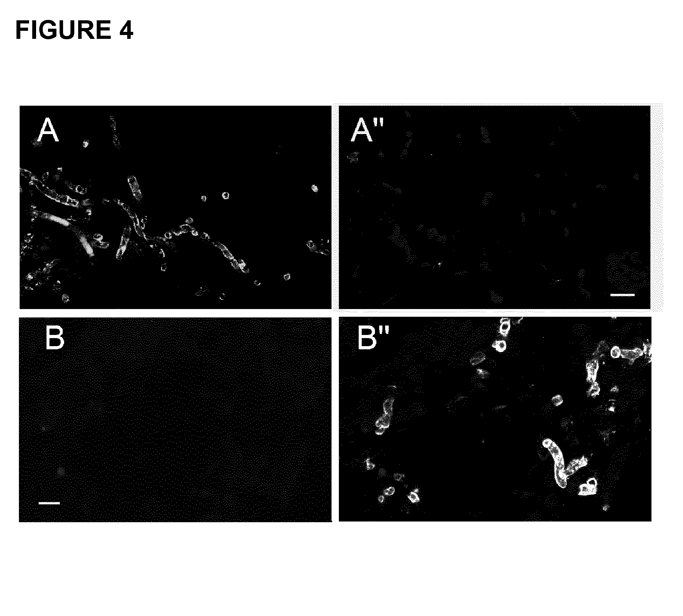

**FIGURE 4****: Immuno-histologic differentiation of** *Aspergillus* ***fumigatus* and *Fusarium solani.***
   Mouse tissue infected with *A. fumigatus* (panels A and A") and *F. solani* (panels B and B") were incubated with a fluorescent-labeled antibodies L10-1 (DyeLight 550; panels A and B) and AB135-8 (DyeLight 480; panels A" and B"), respectively. Hyphae of *A. fumigatus* are only detected by L10-1 but not by AB135-8, whereas hyphae of *F. solani* are only detected by AB135-8 but not by L10-1. Scale bars: 5 µm (panels A and A"); 3 µm (panels B and B").

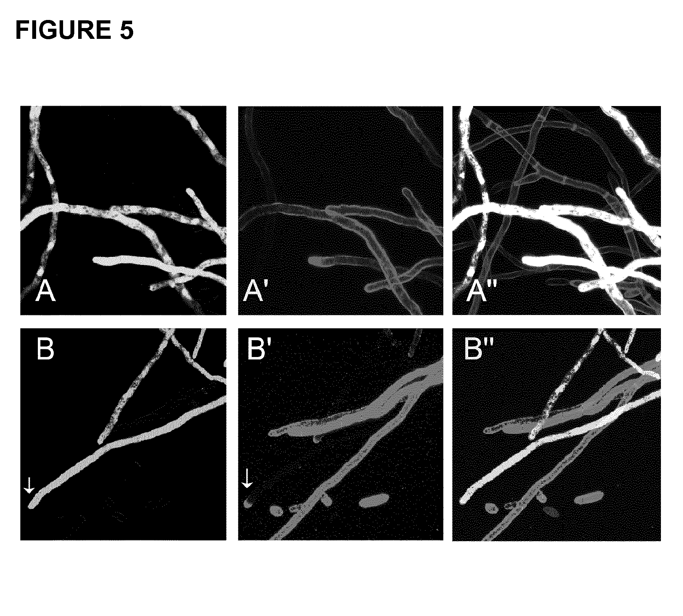

**FIGURE 5****: Double-labeling of hyphae of *Aspergillus fumigatus* ATCC46645-GFP and *Fusarium proliferatum* strain NRZ 2311 using L10-1 and AB135-8.**
   GFP fluorescence of *A. fumigatus* ATCC46645-GFP is shown in panels A and B. Staining with L10-1 and AB135-8 is shown in panels A' and B', respectively. Overlays of GFP- and antibody-derived fluorescence as well as staining of all hyphae with calcofluor white are shown in A" and B". L10-1 specifically detects GFP-expressing *A. fumigatus* hyphae (comparison of A *vs.* A'), whereas AB135-8 labels the entire surface of GFP-negative F. *proliferatum* hyphae (comparison of B, B', and B"). On *A. fumigatus,* AB135-8 detects only the hyphal tips (indicated by an arrow in B'). All panels show projections of several optical planes.

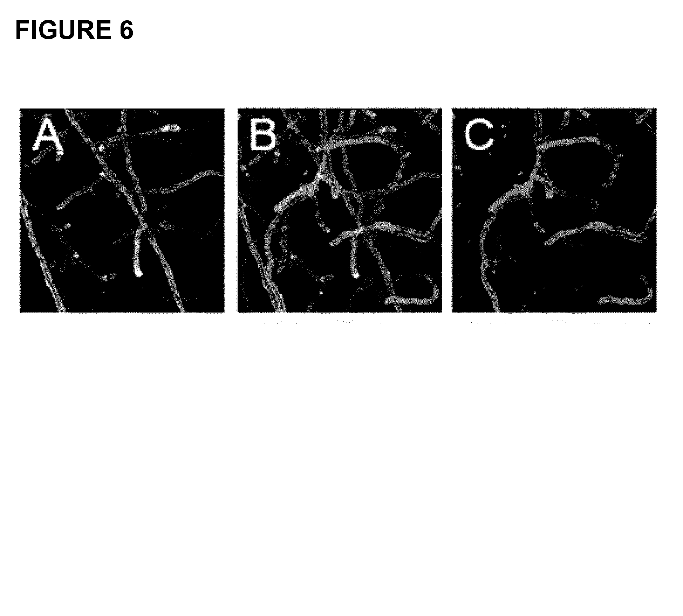

**FIGURE 6****: Differentiation of *Aspergillus fumigatus* and *Fusarium oxysporum* by immunofluorescence.**
   A mixture of hyphae of *A. fumigatus* and *F. oxysporum* was incubated with differentially fluorescent-labeled antibodies L10-1 (DyLight550, red) and AB135-8 (DyLight480, green), respectively. The fluorescent pattern obtained is mutually exclusive. *A. fumigatus* is only detected by L10-1 (panel A), whereas *F. oxysporum* is only detected by AB135-8 (panel C). Panel B shows an overlay of panels A and C.

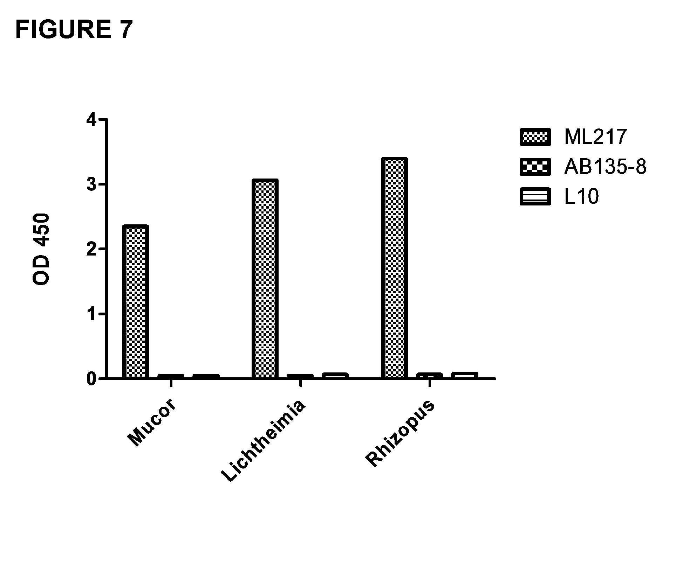

**FIGURE 7****: Antibodies L10-1 and AB135-8 do not recognize zygomycetes.**
   Cell-free supernatants of the zygomycetes *Mucor circinelloides, Lichtheimia corymbifera,* and *Rhizopus oryzae* were diluted 1:100 in coating buffer and coated onto ELISA plates. Plates were blocked and the wells were incubated with the antibodies L10-1, AB135-8 and ML218 (binding to a not yet characterized antigen present in all zygomycetes), respectively. After further washing, wells were incubated with peroxidase-labelled anti-mouse IgM. Enzymatic activity was detected by determining optical density at 450 nm. Only ML218 was recognized by all three species.

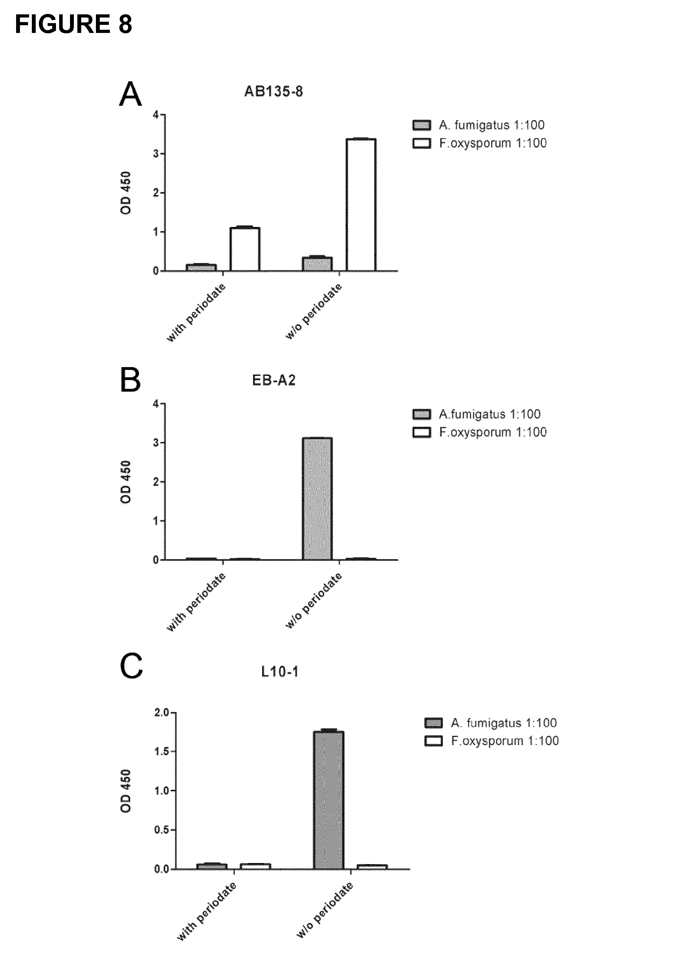

**FIGURE 8****: Sensitivity of galactofuranose antigens to periodate treatment.**
   Cell-free supernatants of *Aspergillus fumigatus* and *Fusarium oxysporum* were coated onto ELISA plates, and incubated with 80 mM periodate overnight. Plates were blocked and incubated with the antibodies AB135-8, EB-A2, and L10-1 respectively. After f washing, wells were incubated with peroxidase-labelled anti-mouse IgM. Enzymatic activity was detected by determining optical density at 450 nm. The β-1,5-Gal*f*ₙ galactomannan antigen (recognized by L10-1 and EB-A2) is highly sensitive to periodate treatment (grey columns in panels B and C), whereas the antigen recognized by AB135-8 shows sensitivity (as expected for a glycoantigen), but is still detectable in the treated samples (white column in panel A). Experiments were performed in triplicate and shown as mean ± SEM.

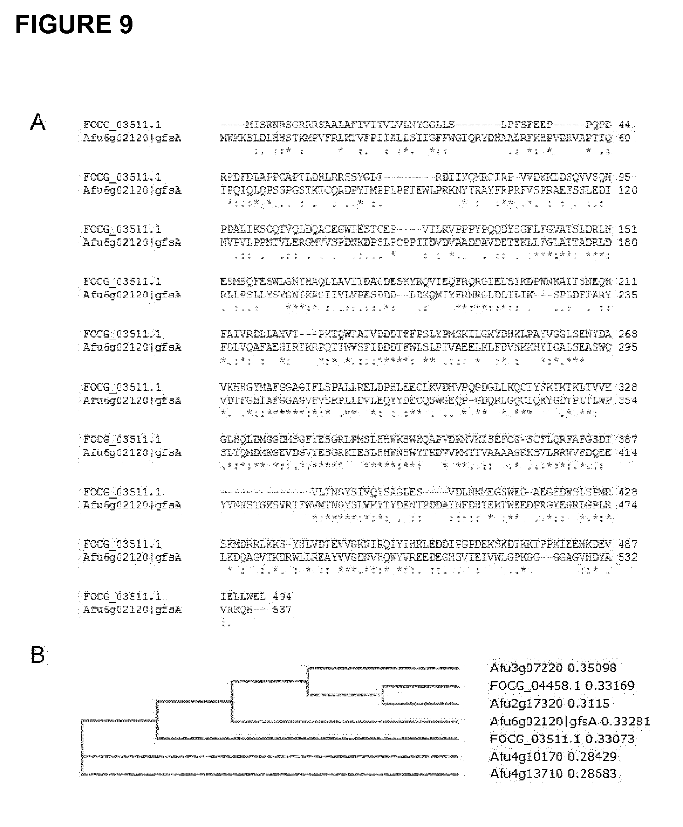

**FIGURE 9****: *Fusarium* protein homologs of *Aspergillus* galactofuranosyltransferase.**
   Panel A: Alignment of the protein sequence being homologous to the *A. fumigatus* galactofuranosyltransferase GfsA (Afu6g02120). Asterisks indicate identical amino acids, dots similar residues. Panel B: Phylogram of GfsA, four homologous *A. fumigatus* proteins and two homologous proteins from *F. oxysporum* strain CL57. Branch lengths are indicated.

### DETAILED DESCRIPTION OF THE INVENTION

The present invention is based on the unexpected identification of a galactofuranose Galf antigen in *Fusarium* that is distinct from the β-1,5-Gal*f*ₙ antigen present in *Aspergillus* and that thus represents a new candidate biomarker for the diagnosis of infections caused by *Fusarium.* This finding was corroborated by the corresponding generation of monoclonal antibody AB135-8 specifically recognizing the Galf antigen in *Fusarium,* thus providing for a superior molecular tool for the easy detection of *Fusarium spec.* and its reliable differentiation from *Aspergillus spec.*

The present invention will be described in the following with respect to particular embodiments and with reference to certain drawings but the invention is to be understood as not limited thereto but only by the appended claims. The drawings described are only schematic and are to be considered non-limiting.

Where the term "comprising" is used in the present description and claims, it does not exclude other elements or steps. For the purposes of the present invention, the term "consisting of" is considered to be a preferred embodiment of the term "comprising". If hereinafter a group is defined to comprise at least a certain number of embodiments, this is also to be understood to disclose a group, which preferably consists only of these embodiments.

Where an indefinite or definite article is used when referring to a singular noun e.g. "a", "an" or "the", this includes a plural of that noun unless specifically stated otherwise.

In case, numerical values are indicated in the context of the present invention the skilled person will understand that the technical effect of the feature in question is ensured within an interval of accuracy, which typically encompasses a deviation of the numerical value given of ± 10%, and preferably of ± 5%.

Furthermore, the terms first, second, third, (a), (b), (c), and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Further definitions of term will be given in the following in the context of which the terms are used. The following terms or definitions are provided solely to aid in the understanding of the invention. These definitions should not be construed to have a scope less than understood by a person of ordinary skill in the art.

The general techniques referred to herein are well understood and commonly employed using conventional methodology by those skilled in the art, such as, for example, the established methodologies described in Sambrook, J., and Russel, D.W. (2001) Molecular cloning: A laboratory manual (3rd Ed.), Cold Spring Harbor Laboratory Press, Cold Spring Harbor, NY; Ausubel, F.M. et al. (2001) Current Protocols in Molecular Biology, Wiley & Sons, Hoboken, N; Albitar, M. (2007) Monoclonal Antibodies: Methods and Protocols, Humana Press, Totawa, NJ; Kontermann, R.E., and Dübel, S. (2010) Antibody Engineering (2nd Ed.), Springer Verlag, Heidelberg.

In one aspect, the present invention relates to an antibody or functional fragment thereof, which specifically binds to a galactofuranose Galf antigen present in *Fusarium spec.,* wherein the antigen is distinct from the β-1,5-Gal*f*ₙ antigen.

The term "antibody", as used herein, refers to a polypeptide product of B cells within the immunoglobulin class of polypeptides that is able to bind to a specific molecular antigen and is composed of two identical pairs of polypeptide chains, wherein each pair has a heavy chain (about 50-70 kDa) and a light chain (about 25 kDa) and each amino-terminal portion of each chain includes a variable region of about 100 to 130 amino acids and each carboxy-terminal portion of each chain includes a constant region. The antibody may be a naturally occurring antibody, a recombinantly produced antibody or an antibody produced by chemical synthesis. Preferably, an antibody according to the present invention is a recombinantly produced antibody.

The term "functional antibody fragment", as used herein, refers to a portion of an antibody heavy or light chain polypeptide that retains some or all of the binding activity of the antibody from which the fragment was derived. Examples of such functional antibody fragments include *inter alia* Fab fragments, F(ab)₂ fragments, F(ab') fragments, F(ab')₂ fragments, single-chain Fvs (scFv), disulfide-linked Fvs (sdFv), Fd fragments, Fv fragments, diabodies, and minibodies (cf., e.g., Greenfield, E.A. (2014) Antibodies: A Laboratory Manual (2nd Ed.), Cold Spring Harbor Laboratory Press, Cold Spring Harbor, NY).

The term "antigen", as used herein, denotes a target or binding site, which is recognized by an antibody or a functional fragment thereof. More specifically, an antibody recognizes a specific epitope of the antigen. The antigen may be a naturally occurring molecular entity or may be synthetic (chemically synthesized or recombinantly produced).

Within the present invention, the antibody or functional fragment thereof specifically binds to a galactofuranose Gal*f* antigen being present in filamentous fungi of the genus *Fusarium* (Summerell, B.A. et al. (2010) Fungal Div. 44, 3-13). Galactofuranose is a five-membered ring form of galactose that is present in various glyco-conjugates in the cell wall of fungi and protozoans (Tefsen, B. et al. (2012), *supra).*

However, the antigen is distinct from the β-1,5-Gal*f*ₙ antigen, also referred to as galactomannan antigen, which comprises chains of β-1,5-linked galactofuranose moieties (Stynen, D. et al. (1992), *supra)* and is present in *Aspergillus* (Tefsen, B. et al. (2011) *supra).* In other words, the Galf antigen of the present invention is a different entity than the galactomannan antigen and specifically recognized by other antibodies than those recognizing the galactomannan antigen.

In one embodiment, the Gal*f* antigen of the present invention is less sensitive to periodate treatment as compared to the β-1,5-Gal*f*ₙ antigen (e.g., at least two times less sensitive, or at least three times less sensitive, or at least five times less sensitive, or at least eight times less sensitive, or at least tem times less sensitive, or more). In another embodiment, the Gal*f* antigen of the present invention is conserved among different species of *Fusarium.*

The term "specifically binds to", as used herein, refers to the selective binding of an antibody to an antigen, that is, binding that is measurably different from a non-specific interaction. Specific binding can be measured, for example, by determining binding of an antibody compared to binding of a control molecule (e.g., another antibody), which is of similar structure but does not have binding activity for the particular antigen concerned. Suitable experimental techniques included *inter alia* enzyme-linked immunosorbent assay (ELISA), radioimmunoassay (RIA), and BIAcore, all well known to the skilled person. As used herein, the term "specific binding" refers to the binding of an antibody or functional fragment thereof that exhibits a dissociation constant (K_{d}) for its Galf antigen target of at least 10⁻⁴ M, or at least 10⁻⁵ M, or at least 10⁻⁶ M, or at least 10⁻⁷ M, or at least 10⁻⁸ M, or at least 10⁻⁹ M, or at least 10⁻¹⁰ M, or at least 10⁻¹¹ M, or at least 10⁻¹² M, or greater. In specific embodiments, the antibody or functional fragment thereof according to the invention that binds to the Gal*f* antigen present in *Fusarium spec.* has a dissociation constant (K_{d}) of ≤ 1 µM, or ≤ 100 nM, or ≤ 10 nM, or ≤ 1 nM, or ≤ 0.1 nM, or less.

As used herein, the term "specific binding" also refers to the binding of an antibody or functional fragment thereof to its target antigen without substantially binding to any other antigen, that is, the antibody or functional fragment thereof does not show cross-reactivity to non-target antigens. In other words, the affinity of the antibody or functional fragment thereof to its target antigen is higher than the affinity to any non-target antigen. In specific embodiments, the antibody or functional fragment thereof of the present invention has less than 15% cross-reactivity with any other antigen than its target antigen, as defined herein, or less than 10% cross-reactivity with any other antigen than its target antigen, or less than 5% cross-reactivity with any other antigen than its target antigen, or less.

In other specific embodiments, the antibody or functional fragment thereof of the present invention has less than 15% cross-reactivity to the β-1,5-Gal*f*ₙ antigen, or less than 10% cross-reactivity to the β-1,5-Gal*f*ₙ antigen, or less than 8% cross-reactivity to the β-1,5-Gal*f*ₙ antigen, or less than 5% cross-reactivity to the β-1,5-Gal*f*ₙ antigen, or less than 4% cross-reactivity to the β-1,5-Gal*f*ₙ antigen, or less than 3% cross-reactivity to the β-1,5-Gal*f*ₙ antigen, or less than 2% cross-reactivity to the β-1,5-Gal*f*ₙ antigen, or less than 1% cross-reactivity to the β-1,5-Gal*f*ₙ antigen; or less.

The antibody or functional fragment thereof according to the present invention is typically of mammalian origin and may be derived from any mammalian species including *inter alia* mouse, rat, chicken, rabbit, monkey, and human. In one embodiment, the antibody or functional fragment thereof is of murine origin. In another embodiment, antibody or functional fragment thereof is of human origin. The antibody or functional fragment thereof may be native or chimeric, that is, with one or more of the complementarity determining regions (CDRs) within the variable domains of the heavy and light chains being derived from another organism (e.g., from mouse) than the remaining portion of the antibody or functional fragments thereof (e.g., from human). In a specific embodiment, the antibody or functional fragment thereof is humanized, that is, it is derived from a non-human species but its amino acid sequence has been modified to increase similarity to variants produced in humans.

The antibody or functional fragment thereof according to the present invention may be of any type of immune globulin (e.g., IgG, IgA, IgD, IgE, IgM, and IgY). In preferred embodiments, the antibody or functional fragment thereof is an IgM antibody.

Typically, the antibody or functional fragment thereof according to the present invention is mono-specific for the Gal*f* antigen being present in *Fusarium* but the engineering of bi-specific, tri-specific or multi-specific antibodies may be possible as well.

The antibody or functional fragment thereof according to the present invention may be polyclonal, that is, derived from several different immune cells. In preferred embodiments, the antibody or functional fragment thereof according to the present invention is monoclonal, that is made from a single clone of an immune cell. Monoclonal antibodies show a monovalent affinity in that they bind to the same antigen epitope. The skilled person is well aware of various methods for generating monoclonal antibodies.

In a particularly preferred embodiment, the antibody is monoclonal antibody AB135-8 (clone AB135-8-1-8) being produced by the hybridoma cell line having accession number DSM ACC3270 and being deposited by the Ludwig-Maximilians-Universität München on May 7, 2015 under the Budapest Treaty at the *Deutsche Sammlung von Mikroorganismen und Zellkulturen* (Braunschweig, Germany).

Another aspect of the present invention relates to the antibody or functional fragment thereof as defined herein for use in the diagnosis of fungal infections caused by *Fusarium spec.* In specific embodiments, the infections caused by *Fusarium spec.* are infections of plants including *inter alia* crown rot, head blight, and scab on cereal grains (e.g., barley), and the Panama disease of banana. In other specific embodiments, the infections caused by *Fusarium spec.* are human infections including *inter alia* onychomycosis, keratomycosis (e.g., in the cornea), sinusitis, systemic infections in immunocompromised patients, and food-caused mycotoxicosis. The skilled person is well aware of techniques for detecting an infection caused by *Fusarium* (such as an ELISA assay or immunofluorescence analysis of a sample, such as a tissue specimen or body fluid, supposed to be infected).

Yet another aspect of the present invention relates to the use of the galactofuranose Gal*f* antigen being specifically recognized by the antibody or functional fragment thereof as defined herein as biomarker for diagnosing a fungal infection caused by *Fusarium spec.*

In specific embodiments, the galactofuranose Gal*f* antigen of the present invention is used as a biomarker in combination with one or more other antigens in a panel of biomarkers. In a particular embodiment, the Gal*f* antigen of the present invention is used in combination the galactomannan β-1,5-Gal*f*ₙ antigen, for example, in order to provide for a set of biomarkers allowing for a differentiation of *Fusarium* and *Aspergillus.*

In a further aspect, the present invention relates to a method for the identification of *Fusarium spec.* in a sample, comprising:
(i) contacting the antibody or functional fragment thereof as defined herein with a sample supposed to comprise *Fusarium spec.;* and
(ii) determining the binding of the antibody or functional fragment thereof to the corresponding galactofuranose Gal*f* antigen,
wherein detection of binding of the antibody is indicative for the presence of *Fusarium spec.* in the sample.

The term "identification", as used herein, is intended to encompass not only the mere detection of the presence of *Fusarium spec.* but also predictions and likelihood analysis in the sense of a diagnosis. The methods of the present invention are also intended to be used clinically in making decisions concerning treatment modalities, including therapeutic intervention, disease staging, and disease monitoring and surveillance. According to the present invention, an intermediate result for examining the condition of a subject may be provided. Such intermediate result may be combined with additional information to assist a physician, nurse, or other practitioner to diagnose that a subject suffers from such a disease or medical condition. Alternatively, the present invention may be used to detect a medical condition in a subject-derived sample, and provide a doctor with useful information to diagnose that the subject suffers from the disease.

Typically, the methods of the present invention for the identification of *Fusarium spec.* are performed as an *in vitro* or *ex vivo* method.

The term "sample", as used herein, is derived from a eukaryotic organism, for example a plant or a mammal. The samples may be used in unpurified form or subjected to any enrichment or purification step(s) prior to use. The skilled person is well aware of various such purification methods. Plant samples may be derived from any plant species and may include *inter alia* tissue sections, e.g., from roots, caulis, leafs or flowers, extracts of plant tissue, cultures of one or more plant cells or cell-free supernatants of plant cell cultures. Mammalian samples may be derived from any mammalian species such as a mouse, rat, hamster, rabbit, cat, dog, pig, cow, horse or monkey, and preferably human. Such samples may include *inter alia* body tissues (e.g., biopsies or resections) and body fluids, such as blood, sputum, and cerebrospinal fluid. The samples may contain a single cell, a cell population (i.e. two or more cells) or a cell extract derived from a body tissue. In specific embodiments, the sample is a blood sample such as whole blood, plasma, and serum. The term "whole blood", as used herein, refers to blood with all its constituents (i.e. both blood cells and plasma). The term "plasma", as used herein, denotes the blood's liquid medium. The term "serum", as used herein, refers to plasma from which the clotting proteins have been removed.

Typically, the antibody or functional fragment thereof is contacted with the sample to be analyzed by simply mixing. Detection of antibody binding may be performed by any suitable immunological detection protocol available in the art, such as enzyme-linked immunosorbent assay (ELISA), radioimmunoassay, magnetic immunoassay, BIAcore, immunofluorescence microscopy, FACS sorting, and the like. In preferred embodiments, antibody binding is determined by a procedure selected from the group consisting of immunofluorescence microscopy and as enzyme-linked immunosorbent assay. The skilled person is well aware of all these techniques. For detection, one or more labels may be directly attached to the (primary) antibody recognizing the galactofuranose Galf antigen, for example, one or more fluorescent labels. Alternatively, a labeled secondary antibody being specific for the primary antibody may be employed.

Labels that may be used herein include any compound, which directly or indirectly generates a detectable compound or signal in a chemical, physical or enzymatic reaction. Labeling and subsequent detection can be achieved by methods well known in the art (see, e.g., Sambrook, J., and Russel, D.W. (2001), *supra;* and Lottspeich, F., and Zorbas H. (1998) Bioanalytik, Spektrum Akademischer Verlag, Heidelberg, Germany). The labels can be selected *inter alia* from fluorescent labels, enzyme labels, chromogenic labels, luminescent labels, radioactive labels, haptens, biotin, metal complexes, metals, and colloidal gold. All these types of labels are well established in the art and can be commercially obtained from various suppliers. In preferred embodiments, fluorescent labels are employed. In other preferred embodiments, enzyme labels are used. An example of a physical reaction that is mediated by such labels is the emission of fluorescence or phosphorescence upon irradiation. Alkaline phosphatase, peroxidase, β-galactosidase, and β-lactamase are examples of enzyme labels, which catalyze the formation of chromogenic reaction products, and which may be used in the invention.

In a specific embodiment, the method further comprises: (iii) quantifying the level of antibody binding in the sample and comparing to a control level.

For example, in a clinical setting, the determination of an elevated level of antibody binding in the test sample as compared to the control level may be indicative for presence and/or the progression of a fungal infection caused by *Fusarium. Vice versa,* the determination of a decreased level of antibody binding in the test sample as compared to the control level may be indicative for regression or relief of a fungal infection caused by *Fusarium,* for example, as result of a therapeutic regimen. Quantification may be accomplished by analyzing various sample volumes, using an internal standard (such as known amounts of another antibody or other binding moiety), using a known amount of competitor molecule interfering with antibody-antigen recognition. In a specific embodiment, the control level is determined by a statistical method based on the results obtained by previous analyses or derived from a database.

In another specific embodiment, the method of claim 8 or 9, further comprising:
(iv) contacting the sample with a second antibody or a functional fragment thereof, wherein the second antibody is capable of specifically binding to the β-1,5-Gal*f*ₙ antigen; and
(v) determining the binding of the second antibody or functional fragment thereof to the corresponding β-1,5-Gal*f*ₙ antigen,
wherein detection of binding of the second antibody is indicative for the presence of *Aspergillus spec.* in the sample.

The method may further comprise quantifying the level of binding of the second antibody in the sample and comparing to a control level, as outlined above for the antibody or functional fragment thereof according to the invention. The antibody or functional fragment thereof according to the invention and the second antibody may be concomitantly or sequentially (in any order) contacted with the sample to be analyzed. Analogously, determination of binding of the different antibodies may also be performed concomitantly or sequentially in any order. Preferably, the second antibody capable of specifically binding to the β-1,5-Gal*f*ₙ antigen is selected from the group consisting of antibody L10-1 (Heesemann, L. et al. (2011) Int. J. Med. Microbiol. 301, 523-530) and antibody EB-A2 (Stynen, D. et al. (1992), *supra).*

In yet another aspect, the invention relates to a kit-of parts, comprising:
(i) a first antibody or functional fragment thereof as defined herein; and
(ii) a second antibody being capable of specifically binding to the β-1,5-Gal*f*ₙ antigen.

Preferably, the second antibody is selected from the group consisting of antibody L10-1 and antibody EB-A2. The kit may further comprise *inter alia* any one or more of the following: one or more secondary antibodies for detecting the antibodies of (i) and (ii), one or more detectable labels, reagents for performing the detection reactions, and suitable buffers.

In a preferred embodiment, the kit-of-parts is used in a diagnostic assay for differentiating *Fusarium spec.* from *Aspergillus spec.*

In a further particular embodiment, the kit-of-parts is used in an *in vitro* diagnostic assay for identifying *Fusarium spec.* in human samples and/or for differentiating *Fusarium spec.* from *Aspergillus spec.* In yet another particular embodiment, the kit-of-parts is used in a diagnostic assay for identifying *Fusarium spec.* in plant samples and/or for differentiating *Fusarium spec.* from *Aspergillus spec.*

The invention is further described by the figures and the following examples, which are solely for the purpose of illustrating specific embodiments of this invention, and are not to be construed as limiting the claimed subject matter in any way.

### EXAMPLES

### EXAMPLE 1: Materials and Methods

### 1.1 Strains and media

The following strains were used in this study: *Fusarium oxysporum* (DSM 62316), *Fusarium solani* (CBS 131394), *Fusarium dimerum* (NRZ Sch63), *Fusarium proliferatum* (NRZ 2311), *Fusarium andiyazi* (CBS 134430), *Aspergillus fumigatus* ATCC46645 and its GFP-expressing derivative (Meier, A. et al. (2003) Cell. Microbiol. 5, 561-570), *Mucor circinelloides* (CBS 277.49), *Lichtheimia corymbifera* (ATCC 46771), and *Rhizopus oryzae* (DSM 1185). Fungal cultures were grown in Aspergillus Minimal Medium (AMM), yeast glucose medium (YG), and Sabouraud medium, respectively, according to established procedures (see, e.g., Schwienbacher, M. et al. (2005) Med. Mycol. 43, 623-630).

### 1.2 Sequence analysis

Protein sequences of *Aspergillus fumigatus* Af293 were obtained from the *Aspergillus* Genome Database (http://www.aspgd.org/). Genomes searches for *Fusarium* and *Mucorales* were performed using the *Fusarium* Comparative Database at the Broad Institute (http://www.broadinstitute.org/annotation/genome/fusarium_group/MultiHome.html) and the Saccharomyces Genome Database (http://www.yeastgenome.org/cgi-bin/blast-fungal.pl), respectively. Alignments and phylogenetic trees were generated using ClustalW2 (http://www.ebi.ac.uk/Tools/msa/clustalw2).

### 1.3 ELISA assays

The PLATELIA *Aspergillus* EIA was performed according to manufacturer's instructions (BIO-RAD Laboratorie, Marnes-la-Coquette, France). In other assay formats, the peroxidase-labeled antibody EB-A2 from the PLATELIA Aspergillus EIA was used as described. For generation of *Aspergillus fumigatus* supernatant (SD-Asp) 200 ml of SD medium (0.34 g/l yeast nitrogen base, 1 g/l ammonium sulfate, 1 g/l glucose) were inoculated with 1 x 10⁸ resting conidia of *A. fumigatus* strain ATCC46645. For the different *Fusarium* strains, 100 ml Sabouraud medium were inoculated with 1.5 x 10⁴ conidia. After 4 days incubation at 37°C with shaking (140 rpm, Certomat-R, B. Braun, Melsungen, Germany), supernatants were harvested by centrifugation, passed through a 0.2 µm filter and stored in aliquots at -20°C.

Samples were diluted in coating buffer (0.1 M NaHCO₃, pH 9.6) and 100 µl were added per well of the ELISA plates (Greiner Bio-One, Frickenhausen, Germany) and the plates were incubated for 2 h at 37°C. For blocking of unspecific binding sites, plates were incubated with 200 µl per well of 0.1 % BSA in TBS for 1 h at 37°C. The primary antibodies were applied as hybridoma culture supernatants. Peroxidase-labeled anti-mouse IgM antibodies were obtained from Dianova (Hamburg, Germany). For washing, plates were incubated five times with 200 µl per well TBS-T (TBS supplemented with 0.1 % Tween 20). Plates were developed using TMB substrate and Stop solution (Mikrogen Diagnostik, Neuried, Germany) and the optical density at 450 nm was determined using a Tecan Sunrise Reader (Tecan, Mannedorf, Switzerland).

### 1.4 Monoclonal antibodies.

Monoclonal AB135-8 was identified from a pool of hybridoma cells raised by immunization of Balb/c mice with killed *A. fumigatus* germ tubes. The subclass of antibodies was determined as being IgM using the Pierce Rapid Antibody Isotyping Kits (Thermo Fisher Scientific, Braunschweig, Germany). Hybridoma cells were grown in Opti-MEM medium (Life Technologies, Darmstadt, Germany) supplemented with 5% fetal calf serum. Purification of IgM antibodies was performed using the Pierce IgM Purification Kit (Thermo Fisher Scientific, Braunschweig, Germany) according to the manufacturer's instructions.

### 1.5 Immunofluorescence.

The fungi were grown to hyphae on glass cover slips in YG medium. *Aspergillus* hyphae stick to glass, but for all other fungi cover slips with weakly attached hyphae had to be dried overnight in a 50°C incubator. Samples were fixed with 3.7% formaldehyde/PBS for 5 min. For immunofluorescence, samples were incubated with monoclonal antibodies L10-1 and AB135-8 for 30 min at 37°C in a moistened chamber. The cover slips were subsequently washed three times in PBS.

Suitable secondary antibodies labeled with either Cy3 dye (red) or Alexa 488 dye (green; both obtained from Dianova, Hamburg, Germany). For direct staining, purified monoclonal antibodies L10-1 and AB135-8 were dialyzed against PBS and labeled using the Dylight 488 and 550 microscale antibody labeling kits (Thermo Fisher Scientific, Dreieich, Germany). To stain the cell wall samples were incubated with 1 mg/ml calcofluor white (Sigma, Deisenhofen, Germany) for one minute at room temperature. After another washing step, samples were embedded using Vectashield Mounting Medium (Vector Laboratories, Burlingame, CA, USA) and analyzed using a Leica SP-5 confocal laser-scanning microscope (Leica Microsystems, Wetzlar, Germany).

### EXAMPLE 2: Fusarium species harbor genes required for galactomannan synthesis.

Galf is the characteristic component of fungal galactomannan. In *Aspergillus fumigatus,* synthesis of Galf requires the UDP-Gal*f* mutase GlfA (Schmalhorst, P.S. et al. (2008), *supra)* and the UDP-galactofuranose GlfB (Engel, J. et al. (2009), *supra). Fusarium solani, F. oxysporum* and *F. verticilloides* were searched for homologous protein sequences using BlastP **(****FIG. 9****).** Conserved proteins were identified in all three *Fusarium* species. *glfA* and *glfB* are neighboring genes in *A. fumigatus* and the same genetic organization was found in *Nectria haematococca,* the teleomorph of *Fusarium solani* (Tefsen, B. et al. (2011), *supra).* Homologous genes of *glfA* and *glfB* are also adjacent in *F. oxysporum* and *F. verticilloides* (data not shown). Thus, *Fusarium* species are apparently able to produce and transport UDP-Gal*f*.

In the Golgi apparatus of *A. fumigatus,* a set of galactofuranosyl-transferases is supposed to incorporate Galf into larger structures, such as glycoproteins, glycolipid or integral cell wall components. So far, only one of these transferases, GsfA, has been identified, which exhibits β-1,5- or β-1,3-galactofuranosyltransferase activity. A search in the *Aspergillus* Genome Database using the GsfA sequence of *A. fumigatus* (AFUA_6G02120) revealed four homologous sequences in this species (AFUA_3G07220, AFUA_2G17320, AFUA_4G10170, and AFUA_4G13710). For all four sequences, conserved genes have been found in several *Fusarium* species. Two sequences from *F. oxysporum* strain CL57 were used for further analysis. An alignment of *A. fumigatus* GsfA and FOCG_0311.1 is shown in **FIG. 9A****,** while **FIG. 9B** depicts a phylogram of all five putative *A. fumigatus* galactofuranosyltransferases and the two homologous sequences of *F. oxysporium* CL57.

### EXAMPLE 3: Detection of a Galf antigen in Fusarium culture supernatants.

Although the standard PLATELIA assay for the detection of *Aspegillus spec.* was initially described to show no reactivity with *Fusarium spec.* (Swanink, C.M. et al. (1997) J. Clin. Microbiol. 35, 257-260) recent studies reported cases of *Fusarium* infections that were associated with positive galactomannan (β-1,5-Gal*f*ₙ) antigen tests.

To examine whether *Fusarium* produces only small amounts of galactomannan, supernatants of fungal cultures in dilutions of 1:2, 1:10, and 1:100 were compared. All *Fusarium* supernatants diluted only 1:2 were positive in the PLATELIA Aspergillus EIA indicating that monoclonal antibody EB-A2 employed in the PLATELIA Aspergillus EIA, is able to apparently detect galactomannan in *Fusarium* culture supernatants. However, the β-1,5-Gal*f*ₙ antigen is produced by *Fusarium* in smaller amounts as compared to *Aspergillus* (cf. also Tortorano, A.M. et al. (2012), *supra).*

An alternative galactomannan-specific monoclonal antibody L10-1 was previously developed (Heesemann, L. et al. (2011) *supra).* Since L10-1 and EB-A2 may have slightly different specificities, L10-1 was used to analyze *Fusarium* supernatants for binding to the β-1,5-Gal*f*ₙ antigen. ELISA wells were coated with purified L10-1, incubated with undiluted *Fusarium* and *Aspergillus* supernatants and developed using peroxidase-coupled EB-A2. Positive signals were obtained for both, *Aspergillus* and *Fusarium* supernatants, demonstrating that L10-1 is able to bind *Fusarium-*derived the β-1,5-Gal*f*ₙ antigen (not shown). L10-1 recognizes *A. fumigatus* hyphae in immunofluorescence, but with hyphae of *F. dimerum, F. solani, F. andiyazi* and *F. oxysporum* no positive staining could be observed (data not shown, compare **FIG. 5****).** Hence, L10-1 detects the galactomannan antigen in low amounts in *Fusarium* culture supernatants, but not on the surface of *Fusarium* hyphae.

### EXAMPLE 4: Monoclonal antibody AB135-8 recognizes a novel galactomannan-related Galf antigen.

Monoclonal antibody AB135-8 derives from a set of hybridoma cell lines initially identified based on their reactivity with *A. fumigatus* germ tubes in immunofluorescence. AB135-8 was particular interesting due to its much stronger reactivity with *Fusarium* hyphae. AB135-8 bound to the hyphae of all five *Fusarium* species tested (i.e., *F. oxysporum, F. solani, F. dimerum, F. proliferatum,* and *F. andiyazi).* No difference was detectable between short and long hyphae. Macroconidia were recognized, but the staining was often patchy **(****FIG. 1B****).** In contrast, microconidia that emerge from hyphae were not recognized by AB135-8 except for small cup-like structures at their base **(****FIG. 1 D)****.** For *A. fumigatus* hyphae, binding was much weaker and in general more focused at the apical parts of hyphae **(****FIG. 2A****).**

Interestingly, AB135-8 did not recognize hyphae of the *A. fumigatus* Δ*glfA* mutant, while a positive staining, similar to wild type, could be observed for a complemented Δ*glfA* mutant **(****FIG. 2B** and **2C****).** Thus, AB135-8 seems to recognize an antigen containing or at least depending on Gal*f*. A carbohydrate antigen is also suggested by the fact that AB135-8 belongs to the IgM subclass of immune globulins (data not shown).

The absence of the "AB135-8" antigen on Δ*glfA* mutant hyphae suggests that Galf is an essential part of the epitope or alternatively that the AB135-8 antigen is anchored to the galactomannan of the cell wall. To distinguish between these two possibilities culture supernatants of the Δ*glfA* mutant and control strains were analyzed. Both, galactomannan and the "AB135-8" antigen were not detectable in supernatants of the mutant (data not shown), but both antigens are released by the parental and complemented strains. These observations strongly suggest that Galf is an essential part of the AB135-8 antigen.

The β-1,5-Gal*f*ₙ galactomannan antigen was not found in supernatants of the *Mucorales* (Cummings, J.R. et al. (2007), *supra),* another major group of fungal pathogens **(****FIG. 7****).** In line with these data, no proteins with significant homologies to GlfA and GlfB could be identified in genomes of several *Mucorales* species (data not shown). In immunofluorescence, AB135-8 showed no binding to hyphae of major *Mucorales* pathogens *Mucor circinelloides, Lichtheimia corymbifera* and *Rhizopus oryzae* (data not shown), which provides further support for the notion that the AB135-8 antigen harbors β-1,5-Gal*f*ₙ.

*Fusarium* hyphae are difficult to distinguish from *Aspergillus* hyphae in light microscopy. Therefore, the ability of L10-1 and AB135-8 antibodies to distinguish hyphae of *A. fumigatus* and *F. oxysporum* was further investigated. To this end, hyphae of *A. fumigatus* expressing cytosolic GFP and *F. oxysporum* were grown as a mixed culture. All hyphae were visualized with Calcofluor white **(****FIG. 5****).** AB135-8 strongly labeled *Fusarium* hyphae, whereas staining of *A. fumigatus* hyphae was more restricted to point-like structure at the apical tip **(****FIG. 5B****').** This may reflect a different distribution of the antigen under different growth conditions or stronger binding of AB135-8 to *Fusarium* hyphae due to a higher density of the antigen. In addition, antibody L10 was labeled with DyLight550 (red), and antibody AB135-8 with DyLight488 (green). Staining of a mixture of hyphae with these directly labeled antibodies also allowed a clear discrimination of *A. fumigatus* and *F. oxysporum* **(****FIG. 6****).**

Specific antibodies are valuable tools for immunohistology. In this study, we analyzed samples from mice infected with *F. solani* and *A. fumigatus.* **(****FIG. 4****)** AB135-8 clearly detected *Fusarium* hyphae **(****FIG. 4B****"),** but showed no reactivity with *A. fumigatus* **(****FIG. 4A****").** L10-1 recognized *A. fumigatus* hyphae **(****FIG. 4A****),** but did not recognize *F. solani.* A mixture of both antibodies led to distinct staining pattern for *A. fumigatus* and *F. solani* that allowed a clear differentiation of both pathogens. Similar results were obtained with tissue samples infected with *F. proliferatum* and *F. oxysporum* (data not shown).

Diagnostics of fungal infections largely depends of the serological detection of certain fungal antigens, such as β-1,3-glucan or galactomannan. However, a specific serological test for *Fusarium* is still lacking. Hence, the potential applicability of AB135-8 for this purpose was analyzed. Culture supernatants of *A. fumigatus* and five *Fusarium* species were incubated with antibodies L10-1, EB-A2, and AB135-8, respectively. Supernatants were diluted 1:100 and coated to ELISA plates. AB135-8 clearly recognized the supernatants of all five *Fusarium* species. In contrast, only weak signals were obtained with *A. fumigatus.* EB-A2 and L10-1 showed the opposite pattern, being strongly positive for *A. fumigatus,* with no or only very weak signals detectable with *Fusarium* supernatants.

Subsequently, it was determined whether the "AB135-8 Gal*f*" antigen resides on the same molecules as the β-1,5-Gal*f*ₙ (galactomannan) antigen recognized by EB-A2. For this purpose, a capture ELISA format was employed using AB135-8 coated on plates and peroxidase-labeled EB-A2 for detection. In this assay, undiluted *A. fumigatus* culture supernatants resulted strong signals, whereas supernatants diluted 1:100 were negative. For *F. oxysporum,* no significant signals even for undiluted culture supernatants could be detected. These results suggest that the two Galf antigens recognized by AB135-8 and EB-A2 are distinct and exist on one molecule, but only in *Aspergillus* not in *Fusarium* supernatants

The present invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by embodiments and optional features, modifications and variations of the inventions embodied therein may be resorted to by those skilled in the art, and that such modifications and variations are considered to be within the scope of this invention.

The invention has been described broadly and generically herein. Each of the narrower species and sub-generic groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.

## Claims

1. Antibody or functional fragment thereof, which specifically binds to a galactofuranose Gal*f* antigen present in *Fusarium spec.,* wherein the antigen is distinct from the β-1,5-Gal*f*ₙ antigen.

2. The antibody or functional fragment thereof of claim 1, wherein the antibody has less than 5% cross-reactivity to the β-1,5-Gal*f*ₙ antigen.

3. The antibody or functional fragment thereof of claim 1 or 2, wherein the antibody is a monoclonal antibody.

4. The antibody or functional fragment thereof of any one of claims 1 to 3, wherein the antibody is an IgM antibody.

5. The antibody of claim 1, wherein the antibody is monoclonal antibody AB135-8 being produced by the hybridoma cell line having accession number DSM ACC3270.

6. The antibody or functional fragment thereof as defined in any one of claims 1 to 5 for use in the diagnosis of fungal infections caused by *Fusarium spec.*

7. Use of the galactofuranose Galf antigen being specifically recognized by the antibody or functional fragment thereof as defined in any one of claims 1 to 5 as biomarker for diagnosing a fungal infection caused by *Fusarium spec.*

8. Method for the identification of *Fusarium spec.* in a sample, comprising:
(i) contacting the antibody or functional fragment thereof as defined in any one of claims 1 to 5 with a sample supposed to comprise *Fusarium spec.;* and
(ii) determining the binding of the antibody or functional fragment thereof to the corresponding galactofuranose Gal*f* antigen,
wherein detection of binding of the antibody is indicative for the presence of *Fusarium spec.* in the sample.

9. The method of claim 8, further comprising:
(iii) quantifying the level of antibody binding in the sample and comparing to a control level.

10. The method of claim 8 or 9, further comprising:
(iv) contacting the sample with a second antibody or a functional fragment thereof, wherein the second antibody is capable of specifically binding to the β-1,5-Gal*f*ₙ antigen; and
(v) determining the binding of the second antibody or functional fragment thereof to the corresponding β-1,5-Gal*f*ₙ antigen,
wherein detection of binding of the second antibody is indicative for the presence of *Aspergillus spec.* in the sample.

11. The method of claim 10, wherein the second antibody is selected from the group consisting of antibody L10-1 and antibody EB-A2.

12. The method of any one of claims 8 to 11, wherein antibody binding is determined by a procedure selected from the group consisting of immunofluorescence microscopy and enzyme-linked immunosorbent assay.

13. Kit-of parts, comprising:
(i) a first antibody or functional fragment thereof as defined in any one of claims 1 to 5; and
(ii) a second antibody being capable of specifically binding to the β-1,5-Gal*f*ₙ antigen.

14. The kit-of-parts of claim 13, wherein the second antibody is selected from the group consisting of antibody L10-1 and antibody EB-A2.

15. Use of the kit-of-parts of claim 13 or 14 in a diagnostic assay for differentiating *Fusarium spec.* from *Aspergillus spec.*
